# EUROPÄISCHE PATENTSCHRIFT

(11) **EP 1 274 405 B1**
(45) Veröffentlichungstag und Bekanntmachung des Hinweises auf die Patenterteilung: **02.06.2004**
(21) Anmeldenummer: 01929488.3
(22) Anmeldetag: 02.04.2001
(51) Int. Cl.: A61K 9/70, A61K 31/465, A61P 25/34

(54) **TRANSDERMALE ODER TRANSMUCOSALE DARREICHUNGSFORMEN MIT EINER NICOTINHALTIGEN WIRKSTOFFKOMBINATION ZUR RAUCHERENTWÖHNUNG**
TRANSDERMAL OR TRANSMUCOSAL DOSAGE FORMS WITH A NICOTINE-CONTAINING ACTIVE SUBSTANCE COMBINATION FOR SMOKER DISINTOXICATION
FORMES GALENIQUES TRANSDERMIQUES OU TRANSMUCOSIQUES COMPRENANT UNE COMBINAISON DE PRINCIPES ACTIFS CONTENANT DE LA NICOTINE, POUR DESINTOXICATION DE FUMEURS

(30) Priorität: 15.04.2000 DE 10018834
(43) Veröffentlichungstag der Anmeldung: 15.01.2003
(73) Patentinhaber: LTS Lohmann Therapie-Systeme AG, 56626 Andernach (DE)
(72) Erfinder: THEOBALD, Frank, 53498 Bad Breisig (DE); FRICK, Ulrich, 56291 Pfalzfeld (DE)
(74) Vertreter: Flaccus, Rolf-Dieter, Dr.
(86) Internationale Anmeldenummer: PCT/EP2001/003712
(87) Internationale Veröffentlichungsnummer: WO 2001/080837

(56) Entgegenhaltungen:
- WO-A-00/16762
- WO-A-96/00072
- CHEMICAL ABSTRACTS, vol. 133, no. 16, 16. Oktober 2000 (2000-10-16) Columbus, Ohio, US; abstract no. 227747n, Seite 1120; XP002155650 & CN 1 239 656 A (ZHENG ET AL.) 29. Dezember 1999 (1999-12-29)
- DATABASE BIOSIS [Online] BIOSCIENCES INFORMATION SERVICE, PHILADELPHIA, PA, US; prev199900198944, D. JORENBY ET AL.: "a controlled trial of sustained-release bupropion, a nicotine patch, or both for smoking cessation" XP002180625 & NEW ENGLAND JOURNAL OF MEDICINE, Bd. 340, Nr. 9, 4. März 1999 (1999-03-04), Seiten 685-691,

## Beschreibung

Die Erfindung betrifft transdermale oder transmucosale pharmazeutische Darreichungsformen zur Behandlung der Nicotinabhängigkeit oder zur Raucherentwöhnung, mit einem Gehalt an Nicotin, einem Nicotinsalz, einem Nicotinderivat oder einem Stoff mit nicotinerger Wirkung, in Kombination mit einem weiteren Wirkstoff.

Die Erfindung betrifft ferner die Verwendung von Nicotin bzw. seiner Salze oder Derivate zur Herstellung von transdermalen oder transmucosalen Arzneiformen zur Behandlung der Nikotinabhängigkeit zum Zwecke der Nicotinsubstitution oder zur Raucherentwöhnung.

Obwohl die gesundheitsschädlichen Auswirkungen des Tabakrauchens allgemein bekannt sind, ist für die meisten Nikotinabhängigen eine Beendigung der Abhängigkeit nur schwer möglich. Der Hauptgrund dafür liegt in den Nicotin-Entzugserscheinungen, welche sich nach Beendigung des Tabakrauchkonsums einstellen. Der Ausstieg aus dieser Suchtabhängigkeit wird deshalb erleichtert, wenn der Nicotinbedarf zumindest während einer Entwöhnungsphase auf andere Weise gedeckt wird, z. B. im Rahmen einer Nicotin-Substitutionstherapie. Dies kann beispielsweise mittels nicotinhaltiger transdermaler therapeutischer Systeme, sogenannter Nicotinpflaster, erfolgen. Diese sind in der Lage, Nicotin über die Haut an den menschlichen Organismus abzugeben, so daß ein Plasmaspiegel aufgebaut wird, durch welchen das Entstehen von Nicotin-Entzugserscheinungen unterdrückt werden kann. Dadurch wird die Raucherentwöhnung erleichtert.

Es hat sich jedoch gezeigt, daß bei vielen Rauchern neben der wirkstoffbezogenen, d. h. nicotinbezogenen, physischen Abhängigkeit zusätzlich eine psychische Abhängigkeit vorliegt, welche durch Nicotinsubstitution alleine nicht behandelt werden kann. Vielfach ist die psychische Abhängigkeit verantwortlich für das Auftreten von Rückfällen.

In diesem Zusammenhang ist es erwähnenswert, daß sich in zahlreichen klinischen Studien gezeigt hat, daß insbesondere die Kombination von Nicotin mit einem Antidepressivum die Erfolgsraten bei der Raucherentwöhnung verbessern kann. Der Erfolg einer Raucherentwöhnungs-Therapie hängt vermutlich zumindest teilweise von der Therapie der psychischen Abhängigkeit ab. Allerdings ist die unterstützende Verabreichung von Psychopharmaka wegen des Nebenwirkungsrisikos und der Gefahr von Über- bzw. Unterdosierungen nicht unproblematisch.

Aus WO 96/00072 sind transdermale und transmucosale Arzneimittelzusammensetzungen bekannt, welche Nicotin in Kombination mit Coffein oder Coffeinderivaten enthalten, und welche zur Raucherentwöhnung eingesetzt werden können. Es wird vermutet, daß eine solche Kombination bei ausstiegswilligen Rauchern auf verbesserte Akzeptanz stößt. Durch die genannte Kombination soll zusätzlich die während der Raucherentwöhnung häufig auftretende Gewichtszunahme verhindert werden. Allerdings kann Coffein nichts zur Überwindung der psychischen Abhängigkeit beitragen. Vielmehr ist zu befürchten, daß die durch Coffein verursachten Nebenwirkungen, wie Reizbarkeit, Nervosität oder Muskeltremor, das Rückfallrisiko erhöhen.

Es war deshalb die Aufgabe der vorliegenden Erfindung, nicotinhaltige pharmazeutische Darreichungsformen bereitzustellen, welche gleichzeitig die Verabreichung eines zusätzlichen Wirkstoffs, vorzugsweise eines Antidepressivums, zur Bekämpfung der psychischen Abhängigkeit im Rahmen einer Raucherentwöhnungs-Therapie ermöglichen. Bei der Verabreichung dieses zusätzlichen Wirkstoffs sollten Nebenwirkungen weitgehend ausgeschlossen werden, und die Anwendung sollte für den Patienten auf einfache und zuverlässige Weise erfolgen können.

Überraschenderweise gelingt die Lösung der Aufgabe durch eine transdermale oder transmucosale pharmazeutische Darreichungsform, welche einen Gehalt an Nicotin, einem Nicotinsalz, einem Nicotinderivat oder einem Stoff mit nicotinerger Wirkung in Kombination mit mindestens einem weiteren Wirkstoff aufweist, wobei die Zubereitung als weitere(n) Wirkstoff(e) mindestens einen auf das Zentralnervensystem wirkenden Stoff enthält. Demgemäß enthalten die erfindungsgemäßen transdermalen oder transmucosalen Darreichungsformen eine Kombination des Wirkstoff Nicotin, oder eines Nicotinsalzes, eines Nicotinderivates oder eines Stoffes mit nicotinerger Wirkung, mit mindestens einem weiteren auf das zentrale Nervensystem wirkenden Stoff.

Mit Hilfe der erfindungsgmmäßen Darreichungsformen ist es möglich, einerseits die für die Nicotinsubstution erforderliche Nicotin-Dosis kontinuierlich über einen bestimmten Zeitraum hinweg an den Patienten zu verabreichen, und gleichzeitig einen auf das Zentralnervensystem wirkenden Stoff, z. B. ein Antidepressivum oder andere Psychopharmaka, zu verabreichen, um die psychische Abhängigkeit zu unterdrücken oder zu bekämpfen. Besonders vorteilhaft ist dabei, daß die Verabreichung auf transdermalem oder auch transmucosalem Wege und mittels eines Kombinationspräparats erfolgt. Dadurch wird vermieden, daß der Patient zur Bekämpfung seiner psychischen Abhängigkeit zusätzliche Arzneimittel oral einnehmen muß. Deshalb ist anzunehmen, daß die Patienten-Compliance durch die Applikation eines einzigen Arzneimittels, welches eine Wirkstoffkombination enthält, verbessert ist.

Die Verabreichung eines auf das Zentralnervensystem wirkenden Stoffs auf transdermalem (oder auch transmucosalem) Wege gestattet es zudem, einen wirksamen Blutplasmaspiegel einzustellen, welcher über den gesamten Applikationszeitraum hinweg aufrecht erhalten werden kann. Dies ist deshalb von Bedeutung, da insbesondere bei zentral wirksamen Substanzen der Erfolg einer Therapie in hohem Maße vom Vorhandensein eines konstanten Plasmaspiegels abhängt. Durch die erfindungsgemäße transdermale oder transmucosale Verabreichung kann das Auftreten unerwünschter Nebenwirkungen - als Folge des Über- bzw. Unterschreitens der therapeutisch wirksamen Dosis - weitestgehend verhindert werden. Ferner wird auf diese Weise auch ein Mißbrauch der darin enthaltenen Wirkstoffe, z. B. Psychopharmaka weitgehend ausgeschlossen.

Zusätzlich ermöglichen die erfindungsgemäßen Darreichungsformen die kontinuierliche, transdermale oder transmucosale Verabreichung von Nicotin oder Nicotinderivaten, so daß ein Nicotin-Blutplasmaspiegel aufgebaut und aufrechterhalten werden kann, welcher für eine Nicotinsubstitution im Rahmen einer Raucherentwöhnungstherapie geeignet ist.

Der angestrebte Nicotin-Blutspiegel liegt im Bereich von 1 ng/ml bis ca. 100 ng/ml, vorzugsweise zwischen 10 ng/ml bis 70 ng/ml, besonders bevorzugt zwischen 10 und 30 ng/ml. Dabei ist zu beachten, daß diese Werte bei einzelnen Patienten unterschiedlich hoch sein können. Dem Fachmann sind die Mittel bekannt, mit denen er das Freisetzungsverhalten von transdermalen oder transmucosalen Darreichungsformen steuern kann, um die gewünschten Werte zu ermöglichen. Dabei ist es vorzuziehen, daß der gewünschte Plasmaspiegel spätestens nach 1 h, vorzugsweise nach spätestens 30 min nach Applikation, erreicht wird.

Bei dem in den erfindungsgemäßen Darreichungsformen zusätzlich zu Nicotin enthaltenen, auf das zentrale Nervensystem wirkenden Stoff handelt es sich um einen Wirkstoff aus der Gruppe der Psychopharmaka, welche die Wirkstoffgruppen der Antidepressiva, Tranquilizer, Nootropika, Neuroleptika, oder Psychomimetika umfaßt. Besonders bevorzugt sind dabei Wirkstoffe aus der Gruppe der Antidepressiva, da sie sich hinsichtlich der Überwindung der psychischen Abhängigkeit als sehr geeignet erwiesen haben. Die Erfindung umfaßt ferner auch nicotinhaltige Darreichungsformen der genannten Art, welche zwei oder mehrere Psychopharmaka aus den genannten Wirkstoffgruppen als zusätzliche Wirkstoffe enthalten.

Insbesondere kann der zusätzliche, auf das Zentralnervensystem wirkende Stoff ausgewählt sein aus der Gruppe, welche die Wirkstoffgruppen der Phenothiazine, Azaphenothiazine, Thioxanthene, Butyrphenone, Diphenylbutylpiperidine, Iminodibenzyl-Derivate, Imonstilben-Derivate, Dibenzocycloheptadien-Derivate, Dibenzodiazepin-Derivate, Dibenzoxepin-Derivate, Benzodiazepine, indol-Derivate, Phenylethylamin-Derivate und Hypericin-Derivate umfaßt.

Ferner schließt die Erfindung insbesondere solche nicotinhaltige transdermale oder transmucosale Darreichungsformen mit ein, welche als zusätzlichen Wirkstoff einen auf das Zentralnervensystem wirkenden Stoff enthalten, der ausgewählt ist aus der Gruppe, welche die Wirkstoffe Chlorpromazin, Perphenazin, Sulpirid, Clozapin, Clomipramin, Trimipramin, Desipramin, Imipramin, Doxepin, Risperidon, Reserpin, Maprotilin, Mianserin, Lofepramin, Tranylcypromin, Moclobemid, Amitriptylin, Paroxetin, Promethazin, Flupentixol, Oxitriptan, Viloxazin, Meprobamat, Hydroxyzin, Buspiron, Fenetyllin, Methylphenidat, Prolintan, Fenfluramin, Fluvoxamin, Meclofenoxat, Nicergolin, Piracetam, Pyritinol, amfebutamon sowie Salze und Derivate dieser Verbindungen enthält.

Als Nicotinsalze bzw. Nicotinderivate können in den erfindungsgemäßen Darreichungsformen vorzugsweise Nicotinhydrochlorid, Nicotindihydrochlorid, Nicotinsulfat, Nicotin-bitartrat, Nicotin-Zinkchlorid und Nicotinsalicylat eingesetzt werden, entweder einzeln oder in Kombination, oder auch in Kombination mit Nicotin.
Als Substanzen mit nicotinerger Wirkung, d. h. Substanzen mit Wirkung am Nicotin-Rezeptor, werden neben Nicotin selbst bevorzugt Lobelin, Succinylcholin und andere periphere Muskelrelaxantien eingesetzt.

Die für eine Behandlung der psychischen Abhängigkeit geeigneten Wirkstoffdosen und Plasmaspiegel sind dem Fachmann bekannt. Vorzugsweise wird die Dosis des auf das Zentralnervensystem wirkenden Stoffes auf die in der Darreichungsform vorhandene Nicotindosis abgestimmt, derartig, daß beide Wirkstoffe möglichst den jeweils therapeutisch günstigen Plasmaspiegel aufbauen und über eine vorherbestimmbare Applikationsdauer eine gleichmäßig anhaltende Wirkstofffreisetzung gewährleisten.

Eine Gruppe von Ausführungsformen der Erfindung sieht vor, daß die transdermalen Darreichungsformen als transdermale therapeutische Systeme (TTS) gestaltet sind, welche als Wirkstoffpflaster auf die Haut des Patienten geklebt werden können und danach die Wirkstoffe über die Haut freisetzen. Der Aufbau eines solchen Systems weist im allgemeinen eine wirkstoffundurchlässige Rückschicht, ein mit dieser verbundenes, vorzugsweise haftklebendes wirkstoffhaltiges Reservoir und eine vor der Applikation ablösbare Schutzschicht auf. Das wirkstoffhaltige Reservoir enthält eine Kombination aus mindestens zwei Wirkstoffen, nämlich Nicotin, ein Nicotinsalz, ein Nicotinderivat oder einen Stoff mit nicotinerger Wirkung, sowie als weitere wirkstoffkomponente(n) zusätzlich mindestens einen auf das Zentralnervensystem wirkenden Stoff, welcher aus den oben genannten Stoffen oder Stoffgruppen ausgewählt werden kann.

Das Wirkstoffreservoir des TTS, welches die Wirkstoffe in gelöster oder dispergierter Form enthält, ist im wesentlichen auf der Basis von Polymeren aufgebaut, welche eine Wirkstoffmatrix bilden; diese Polymere werden auch als Basispolymere bezeichnet. Das Wirkstoffreservoir besitzt vorzugsweise haftklebende Eigenschaften, welche durch die Art der verwendeten Basispolymere oder durch zusätzlich beigemengte klebrigmachende Stoffe bedingt sind.

Ferner ist es auch möglich, das Wirkstoffreservoir auf der Hautseite mit einer haftklebenden Schicht auszustatten. Das Wirkstoffreservoir der erfindungsgemäßen TTS ist in der einfachsten Ausführungsform eine einschichtige Polymermatrix. Abweichend davon kann das TTS einen schichtfönnigen Aufbau des Wirkstoffreservoirs mit mindestens zwei Polymer-Matrixschichten aufweisen. Dabei kann es vorteilhaft sein, wenn zwei Polymerschichten hinsichtlich ihres Wirkstoffgehalts unterscheiden. Beispielsweise kann eine der wirkstoffhaltigen Matrixschichten des Wirkstoffreservoirs Nicotin als Wirkstoff enthalten, und eine zweite Matrixschicht ein Antidepressivum als erfindungsgemäß vorgesehenen zweiten Wirkstoff.

Als Basis-Polymere der erfindungsgemäßen TTS werden vor allem Acrylsäureester enthaltende Copolymere eingesetzt, darüber hinaus auch Mischungen aus Kautschuken und Harzen, Synthesekautschuke, Polyvinylacetat, Polyvinylpyrrolidone, Silikonpolymere, Cellulosederivate, Heißschmelzkleber und viele andere Materialien, deren Verwendung auf der menschlichen Haut unbedenklich ist. Es können auch Mischungen verschiedener Basispolymere eingesetzt werden.

Die wirkstoffhaltige Polymermatrix kann zusätzlich Hilfsstoffe und Zusatzstoffe enthalten, insbesondere hautpermeationsfördernde Zusatzstoffe, die dem Fachmann bekannt sind.

Der Aufbau der erfindungsgemäßen TTS umfaßt neben dem oben besprochenen Wirkstoffreservoir eine wirkstoffundurchlässige Rückschicht sowie eine ebenfalls wirkstoffundurchlässige ablösbare Schutzfolie. Als Material für die Rückschicht eignen sich eine Vielzahl von hautverträglichen Kunststoffolien, wie z. B. Folien aus Polyvinylchlorid, Ethylenvinylacetat, Vinylacetat, Polyethylen, Polypropylen oder Cellulosederivaten. Besonders geeignet als Material für die Rückschicht sind Polyester, die sich durch besondere Festigkeit auszeichnen. Ferner kann es im Einzelfall nützlich sein, die aus Folienmaterial bestehende Rückschicht mit einer zusätzlichen Auflage zu versehen, z. B. durch Bedampfung mit Metallen oder anderen diffusionssperrenden Zusatzstoffen wie Siliciumdioxid, Aluminiumoxid oder ähnlicher, dem Fachmann bekannter Stoffe.

Für die ablösbare Schutzschicht der TTS können grundsätzlich dieselben Materialien verwendet werden wie für die Rückschicht, vorausgesetzt, daß diese Schicht einer geeigneten Oberflächenbehandlung, z. B. Fluorosilikonisierung, unterzogen wird, so daß sie von der von ihr bedeckten Haftklebeschicht ablösbar ist und vor Applikation des TTS abgezogen werden kann. Außerdem können als ablösbare Schutzschichten auch andere Materialien verwendet werden, wie z. B. Polytetrafluorethylen-behandeltes Papier, Cellophan, Polyvinylchlorid oder ähnliche.

Die Herstellung der vorstehend beschriebenen TTS kann mit Verfahren erfolgen, welche dem Fachmann bekannt sind. Dabei wird im allgemeinen eine Lösung oder Schmelze der Matrix-Basispolymere hergestellt, in welcher die Wirkstoffe gelöst bzw. homogen dispergiert werden. Anschließend wird die wirkstoffhaltige Masse auf eine folienförmige Unterlage beschichtet, getrocknet und mit einer weiteren Folienschicht bedeckt. Aus dem entstandenen Laminat werden sodann einzelne Wirkstoffpflaster ausgestanzt. Die Fläche der einzelnen Pflaster wird vorzugsweise so bemessen, daß ein Pflaster jeweils einer halben oder einer ganzen Tagesdosis oder einer 2- oder 3-Tagesdosis entspricht, jeweils bezogen auf Nicotin bzw. den Kombinationswirkstoff (z. B. Antidepressivum).

Eine weitere Gruppe von Ausführungsformen der Erfindung sieht vor, daß die Nicotin und mindestens einen auf das Zentralnervensystem wirkenden Stoff enthaltenden Darreichungsformen als transmucosale Darreichungsformen ausgestaltet sind. Solche transmucosale Darreichungsformen können beispielsweise im Mundraum zur sublingualen oder bukkalen Applikation angewandt werden. Die Wirkstoffaufnahme erfolgt dabei über die Schleimhaut unter Umgehung des Verdauungstraktes.

Eine erfindungsgemäße transmucosale Darreichungsform, welche eine Kombination von Nicotin mit einem der vorstehend genannten Wirkstoffe enthält, ist vorzugsweise als flacher, film-, folien- oder oblatenförmiger Wirkstoffträger ausgebildet und mit mucoadhäsiven Eigenschaften ausgerüstet. Dadurch wird das Festhaften der Arzneiform an der Oberfläche während des Applikationszeitraums gewährleistet. Die mucoadhäsiven Eigenschaften können durch Zusatz geeigneter polymerer Hilfsstoffe bewirkt werden, welche vorzugsweise wasserquellbare Eigenschaften haben, beispielsweise Stärke, Carboxymethylcellulose, Hydroxypropylcellulose, Polyacrylsäure, Polyvinylpyrrolidone, Polyethylenoxid-Polymere, Ethyl- oder Propylcellulose, Alginate, Pectine oder natürliche Gummen.

Die erfindungsgemäßen Darreichungsformen vereinen die Vorteile einer Kombinationstherapie, umfassend Nicotin oder ein Nicotinsalz, ein Nicotinderivat oder einen Stoff mit nicotinerger Wirkung zur Substitutionstherapie und einen auf das Zentralnervensystem wirkenden Stoff zur Behandlung der psychischen Abhängigkeit, mit den Vorteilen der transdermalen bzw. transmucosalen Verabreichung. Aus diesen Gründen können sie vorteilhaft zur Behandlung der Nicotinabhängigkeit, zur Raucherentwöhnung bzw. zur Nicotinsubstitution eingesetzt werden. Anders als bekannte nicotinhaltige Wirkstoffpflaster ermöglichen die erfindungsgemäßen Darreichungsformen nicht nur eine Nicotinsubstitution, sondern sie gestatten gleichzeitig eine Behandlung der psychischen Abhängigkeitskomponente der Nicotinsucht.

Vorzugsweise werden die erfindungsgemäßen Darreichungsformen verwendet, um Nicotin bzw. eine dem Nicotin verwandte Substanz in Kombination mit einem geeigneten Antidepressivum, oder einer anderen zur Behandlung der psychischen Abhängigkeit geeigneten, auf das Zentralnervensystem wirkenden Substanz, auf transdermalem oder transmucosalem Wege an Patienten im Rahmen einer Behandlung zur Raucherentwöhnung zu verabreichen.

## Patentansprüche

1. Transdermale oder transmucosale pharmazeutische Darreichungsform zur Behandlung der Nicotinabhängigkeit oder zur Raucherentwöhnung, mit einem Gehalt an Nicotin, einem Nicotinsalz, einem Nicotinderivat oder einem Stoff mit nicotinerger Wirkung, in Kombination mit mindestens einem weiteren Wirkstoff, **dadurch gekennzeichnet, daß** sie als zusätzliche(n) Wirkstoff(e) mindestens einen auf das Zentralnervensystem wirkenden Stoff enthält, wobei der zusätzliche, auf das Zentralnervensystem wirkende Stoff ausgewählt ist aus der Gruppe der Psychopharmaka, welche die Wirkstoffgruppen der Antidepressiva, Tranquilizer, Nootropika, Neuroleptika oder Psychomimetika umfaßt.

2. Darreichungsform nach Anspruch 1, **dadurch gekennzeichnet, daß** sie ein Nicotinderivat oder ein Nicotinsalz enthält, welches vorzugsweise ausgewählt ist aus der Gruppe, die Nicotinhydrochlorid, Nicotindihydrochlorid, Nicotinsulfat, Nicotin-bitartrat, Nicotin-Zinkchlorid und Nicotinsalicylat umfaßt.

3. Darreichungsform nach Anspruch 1, **dadurch gekennzeichnet, daß** sie einen Stoff mit nicotinerger Wirkung enthält, vorzugsweise ausgewählt aus der Gruppe, die Nicotin, Lobelin, Succinylcholin und andere periphere Muskelrelaxantien umfaßt.

4. Darreichungsform nach einem der vorangehenden Ansprüche, **dadurch gekennzeichnet, daß** der zusätzliche, auf das Zentralnervensystem wirkende Stoff ausgewählt ist aus der Gruppe, welche die Wirkstoffgruppen der Phenothiazine, Azaphenothiazine, Thioxanthene, Butyrphenone, Diphenylbutylpiperidine, Iminodibenzyl-Derivate, Imonstilben-Derivate, Dibenzocycloheptadien-Derivate, Dibenzodiazepin-Derivate, Dibenzoxepin-Derivate, Benzodiazepine, Indol-Derivate, Phenylethylamin-Derivate und Hypericin-Derivate umfaßt.

5. Darreichungsform nach einem der vorangehenden Ansprüche, **dadurch gekennzeichnet, daß** der zusätzliche, auf das Zentralnervensystem wirkende Stoff ausgewählt ist aus der Gruppe, welche die Wirkstoffe Chlorpromazin, Perphenazin, Sulpirid, Clozapin, Clomipramin, Trimipramin, Desipramin, Imipramin, Doxepin, Risperidon, Reserpin, Maprotilin, Mianserin, Lofepramin, Tranylcypromin, Moclobemid, Amitriptylin, Paroxetin, Promethazin, Flupentixol, Oxitriptan, Viloxazin, Meprobamat, Hydroxyzin, Buspiron, Fenetyllin, Methylphenidat, Prolintan, Fenfluramin, Fluvoxamin, Meclofenoxat, Nicergolin, Piracetam, Pyritinol, Amfebutamon sowie Salze und Derivate dieser Verbindungen enthält.

6. Darreichungsform nach einem oder mehreren der vorangehenden Ansprüche, **dadurch gekennzeichnet, daß** sie die transdermale Verabreichung der Wirkstoffe ermöglicht, wobei die Darreichungsform vorzugsweise als transdermales therapeutisches System gestaltet ist, welches eine wirkstoffundurchlässige Rückschicht, ein vorzugsweise haftklebendes, wirkstoffhaltiges Reservoir mit einem Gehalt an Nicotin, einem Nicotinsalz, einem Nicotinderivat oder einem Stoff mit nicotinerger Wirkung sowie zusätzlich mindestens einen auf das Zentralnervensystem wirkenden Stoff, und eine vor der Applikation ablösbare Schutzschicht aufweist.

7. Darreichungsform nach einem oder mehreren der vorangehenden Ansprüche, **dadurch gekennzeichnet, daß** sie die transmucosale Verabreichung der Wirkstoffe ermöglicht, wobei die Darreichungsform vorzugsweise als flacher, film-, folienoder oblatenförmiger Wirkstoffträger ausgebildet und mit mucoadhäsiven Eigenschaften ausgerüstet ist, wobei die mucoadhäsiven Eigenschaften durch einen Zusatz eines polymeren Hilfsstoffes, vorzugsweise aus der Stärke, Carboxymethylcellulose oder Polyacrylsäure umfassenden Gruppe, bewirkt werden.

8. Verwendung von Nicotin, einem Nicotinsalz, einem Nicotinderivat oder einem Stoff mit nicotinerger Wirkung, in Kombination mit mindestens einem auf das Zentralnervensystem wirkenden Stoff, der aus der Gruppe der Psychopharmaka ausgewählt ist, welche die Wirkstoffgruppen der Antidepressiva, Tranquilizer, Nootropika, Neuroleptika oder Psychomimetika umfasst, zur Herstellung eines transdermalen therapeutischen Systems oder einer transmucosalen Darreichungsform zur Behandlung der Nicotinabhängigkeit, zur Raucherentwöhnung oder zur Nikotinsubstitution.

9. Verwendung nach Anspruch 8, **dadurch gekennzeichnet, daß** der auf das Zentralnervensystem wirkende Stoff ausgewählt ist aus der Gruppe, welche die Wirkstoffgruppen der Phenothiazine, Azaphenothiazine, Thioxanthene, Butyrphenone, Diphenylbutylpiperidine, Iminodibenzyl-Derivate, Imonstilben-Derivate, Dibenzocycloheptadien-Derivate, Dibenzodiazepin-Derivate, Dibenzoxepin-Derivate, Benzodiazepine, Indol-Derivate, Phenylethylamin-Derivate und Hypericin-Derivate umfaßt.

10. Verwendung nach Anspruch 8, **dadurch gekennzeichnet, daß** der auf das Zentralnervensystem wirkende Stoff ausgewählt ist aus der Gruppe, welche die Wirkstoffe Chlorpromazin, Perphenazin, Sulpirid, Clozapin, Clomipramin, Trimipramin, Desipramin, Imipramin, Doxepin, Risperidon, Reserpin, Maprotilin, Mianserin, Lofepramin, Tranylcypromin, Moclobemid, Amitriptylin, Paroxetin, Promethazin, Flupentixol, Oxitriptan, Viloxazin, Meprobamat, Hydroxyzin, Buspiron, Fenetyllin, Methylphenidat, Prolintan, Fenfluramin, Fluvoxamin, Meclofenoxat, Nicergolin, Piracetam, Pyritinol, Amfebutamon sowie Salze und Derivate dieser Verbindungen enthält.

## Claims

1. Transdermal or transmucosal pharmaceutical administration form for treating nicotine dependency or for disaccustoming smokers, comprising nicotine, a nicotine salt, a nicotine derivative or a nicotinergic substance in combination with at least one further active substance, **characterized in that** as additional active substance(s) it comprises at least one substance which acts on the central nervous system, said additional substance which acts on the central nervous system being selected from the group of the psychopharmaceuticals which embraces the active-substance groups of the antidepressants, tranquilizers, nootropics, neuroleptics, or psychomimetics.

2. Administration form according to Claim 1, **characterized in that** it comprises a nicotine derivative or a nicotine salt which is preferably selected from the group which encompasses nicotine hydrochloride, nicotine dihydrochloride, nicotine sulphate, nicotine bitartrate, nicotine-zinc chloride and nicotine salicylate.

3. Administration form according to Claim 1, **characterized in that** it comprises a nicotinergic substance selected preferably from the group encompassing nicotine, lobeline, succinylcholine and other peripheral muscle relaxants.

4. Administration form according to any one of the preceding claims, **characterized in that** the additional substance which acts on the central nervous system is selected from the group which embraces the active-substance groups of the phenothiazines, azaphenothiazines, thioxanthenes, butyrophenones, diphenylbutylpiperidines, iminodibenzyl derivatives, iminostilbene derivatives, dibenzocycloheptadiene derivatives, dibenzodiazepine derivatives, dibenzoxepin derivatives, benzodiazepines, indole derivatives, phenylethylamine derivatives and hypericin derivatives.

5. Administration form according to any one of the preceding claims, **characterized in that** the additional substance which acts on the central nervous system is selected from the group containing the active substances chlorpromazine, perphenazine, sulpiride, clozapine, clomipramine, trimipramine, desipramine, imipramine, doxepin, risperidone, reserpine, maprotiline, mianserin, lofepramine, tranylcypromine, moclobemide, amitriptyline, paroxetine, promethazine, flupentixol, oxitriptan, viloxazine, meprobamate, hydroxyzine, buspirone, fenetylline, methylphenidate, prolintane, fenfluramine, fluvoxamine, meclofenoxate, nicergoline, piracetam, pyritinol, amfebutamone, and also salts and derivatives of these compounds.

6. Administration form according to one or more of the preceding claims, **characterized in that** it permits the transdermal administration of the active substances, the administration form preferably being designed as a transdermal therapeutic system which comprises a backing layer which is impermeable to active substance, a preferably pressure-sensitively adhering active substance reservoir containing nicotine, a nicotine salt, a nicotine derivative or a nicotinergic substance and also, in addition, at least one substance which acts on the central nervous system, and a protective layer which can be removed prior to application.

7. Administration form according to one or more of the preceding claims, **characterized in that** it permits the transmucosal administration of the active substances, the administration form preferably being designed as a flat, film-like, foil-like or wafer-like active substance carrier and being provided with mucoadhesive properties, the mucoadhesive properties being brought about by the addition of a polymeric auxiliary, preferably from the group encompassing starch, carboxymethylcellulose and polyacrylic acid.

8. Use of nicotine, a nicotine salt, a nicotine derivative or a nicotinergic substance in combination with at least one substance which acts on the central nervous system and is selected from the group of the psychopharmaceuticals which encompasses the active-substance groups of the antidepressants, tranquillizers, nootropics, neuroleptics, and psychomimetics, for producing a transdermal therapeutic system or a transmucosal administration form for the treatment of nicotine dependency, for smoker disaccustomization, or for nicotine substitution.

9. Use according to Claim 9, **characterized in that** the substance which acts on the central nervous system is selected from the group which embraces the active-substance groups of the phenothiazines, azaphenothiazines, thioxanthenes, butyrophenones, diphenylbutylpiperidines, iminodibenzyl derivatives, iminostilbene derivatives, dibenzocycloheptadiene derivatives, dibenzodiazepine derivatives, dibenzoxepin derivatives, benzodiazepines, indole derivatives, phenylethylamine derivatives and hypericin derivatives.

10. Use according to Claim 8, **characterized in that** the substance which acts on the central nervous system is selected from the group containing the active substances chlorpromazine, perphenazine, sulpiride, clozapine, clomipramine, trimipramine, desipramine, imipramine, doxepin, risperidone, reserpine, maprotiline, mianserin, lofepramine, tranylcypromine, moclobemide, amitriptyline, paroxetine, promethazine, flupentixol, oxitriptan, viloxazine, meprobamate, hydroxyzine, buspirone, fenetylline, methylphenidate, prolintane, fenfluramine, fluvoxamine, meclofenoxate, nicergoline, piracetam, pyritinol, amfebutamone, and also salts and derivatives of these compounds.

## Revendications

1. Forme d'administration pharmaceutique transdermique ou transmuqueuse pour le traitement de la dépendance à la nicotine ou pour le sevrage tabagique, possédant une teneur en nicotine, en un sel de nicotine, en un dérivé de nicotine ou en une substance ayant un effet nicotinergique, en combinaison avec au moins une substance active supplémentaire, **caractérisée en ce qu'**elle contient, à titre de substance(s) active(s) supplémentaire(s), au moins une substance agissant sur le système nerveux central, la substance supplémentaire agissant sur le système nerveux central étant choisie parmi le groupe des psychotropes qui comprend les groupes de substances actives que sont les antidépresseurs, les tranquillisants, les nootropes, les neuroleptiques ou les psychomimétiques.

2. Forme d'administration selon la revendication 1, **caractérisée en ce qu'**elle contient un dérivé de nicotine ou un sel de nicotine qui est de préférence choisi parmi le groupe qui comprend le chlorhydrate de nicotine, le dichlorhydrate de nicotine, le sulfate de nicotine, le ditartrate de nicotine, le chlorure de zinc de nicotine et le salicylate de nicotine.

3. Forme d'administration selon la revendication 1, **caractérisée en ce qu'**elle comprend une substance ayant un effet nicotinergique choisie de préférence parmi le groupe comprenant la nicotine, la lobéline, la succinylcholine et d'autres relaxants musculaires périphériques.

4. Forme d'administration selon l'une quelconque des revendications précédentes, **caractérisée en ce que** la substance supplémentaire agissant sur le système nerveux central est choisie parmi le groupe qui comprend les groupes de substances actives que sont les phénothiazines, les azaphénothiazines, les thioxanthènes, les butyrophénones, les diphénylbutylpipéridines, les dérivés d'iminodibenzyle, les dérivés d'iminostylbène, les dérivés de dibenzocycloheptadiène, les dérivés de dibenzodiazépines, les dérivés de dibenzoxépines, les benzodiazépines, les dérivés d'indole, les dérivés de phényléthylamine et les dérivés d'hypéricine.

5. Forme d'administration selon l'une quelconque des revendications précédentes, **caractérisée en ce que** la substance supplémentaire agissant sur le système nerveux central est choisie parmi le groupe qui contient les substances actives que sont la chloropromazine, la perphénazine, le sulpiride, la clozapine, la clomipramine, la trimipramine, la désipramine, l'imipramine, la doxépine, la rispéridone, la réserpine, la maprotiline, la miansérine, la lofépramine, la tranylcypromine, le moclobémide, l'amitriptyline, la paroxétine, la prométhazine, le flupentixol, l'oxitriptan, la viloxazine, le méprobamate, l'hydroxyzine, le buspiron, la fénétylline, le méthylphénidate, le prolintane, la fenfluramine, la fluvoxamine, le méclofénoxate, la nicergoline, le piracétam, le pyritinol, l'amfébutamone, ainsi que des sels et des dérivés de ces composés.

6. Forme d'administration selon une ou plusieurs des revendications précédentes, **caractérisée en ce qu'**elle permet l'administration transdermique des substances actives, la forme d'administration étant mise en oeuvre de préférence sous la forme d'un système thérapeutique transdermique qui présente une couche dorsale imperméable à la substance active, un réservoir contenant la substance active, de préférence autoadhésif, possédant une teneur en nicotine, en un sel de nicotine, en un dérivé de nicotine ou en une substance ayant un effet nicotinergique, ainsi que, en outre, au moins une substance agissant sur le système nerveux central, et une couche de protection qui peut être retirée avant l'application.

7. Forme d'administration selon une ou plusieurs des revendications précédentes, **caractérisée en ce qu'**elle permet l'administration transmuqueuse des substances actives, la forme d'administration étant mise en oeuvre de préférence sous la forme d'un support de substance active de forme plate, en forme de film, en forme de feuille ou en forme d'hostie et étant équipée de propriétés mucoadhésives, les propriétés mucoadhésives étant mises en oeuvre par l'addition d'une substance auxiliaire polymère, de préférence constituée du groupe comprenant l'amidon, la carboxyméthylcellulose et l'acide polyacrylique.

8. Utilisation de nicotine, d'un sel de nicotine, d'un dérivé de nicotine ou d'une substance ayant un effet nicotinergique, en combinaison avec au moins une substance agissant sur le système nerveux central qui est choisie parmi le groupe des psychotropes qui comprend les groupes de substances actives que sont les antidépresseurs, les tranquillisants, les nootropes, les neuroleptiques ou les psychomimétiques, pour la préparation d'un système thérapeutique transdermique ou d'une forme d'administration transmuqueuse pour le traitement de la dépendance à la nicotine, pour le sevrage tabagique ou pour le remplacement de la nicotine.

9. Utilisation selon la revendication 8, **caractérisée en ce que** la substance agissant sur le système nerveux central est choisie parmi le groupe qui comprend les groupes de substances actives que sont les phénothiazines, les azaphénothiazines, les thioxanthènes, les butyrophénones, les diphénylbutylpipéridines, les dérivés d'iminodibenzyle, les dérivés d'iminostylbène, les dérivés de dibenzocycloheptadiène, les dérivés de dibenzodiazépines, les dérivés de dibenzoxépines, les benzodiazépines, les dérivés d'indole, les dérivés de phényléthylamine et les dérivés d'hypéricine.

10. Utilisation selon la revendication 8, **caractérisée en ce que** la substance agissant sur le système nerveux central est choisie parmi le groupe qui contient les substances actives que sont la chloropromazine, la perphénazine, le sulpiride, la clozapine, la clomipramine, la trimipramine, la désipramine, l'imipramine, la doxépine, la rispéridone, la réserpine, la maprotitine, la miansérine, la lofépramine, la tranylcypromine, le moclobémide, l'amitriptyline, la paroxétine, la prométhazine, le flupentixol, l'oxitriptan, la viloxazine, le méprobamate, l'hydroxyzine, le buspiron, la fénétylline, le méthylphénidate, le prolintane, la fenfluramine, la fluvoxamine, le méclofénoxate, la nicergoline, le piracétam, le pyritinol, l'amfébutamone, ainsi que des sels et des dérivés de ces composés.
